# EUROPEAN PATENT APPLICATION

(11) **EP 2 862 589 A1**
(43) Date of publication of application: **22.04.2015**
(21) Application number: 14188072.4
(22) Date of filing: 08.10.2014
(51) Int. Cl.: A61M 25/00, D04C 1/06

(54) **Catheter with cross-braided proximal section and helical-coiled distal end**

(30) Priority: 09.10.2013 US 201314049373
(71) Applicant: Biosense Webster (Israel), Ltd., 20692 Yokneam (IL)
(72) Inventor: Govari, Assaf, 3440001 Haifa (IL); Beeckler, Christopher Thomas, Brea, California 92821 (US)
(74) Representative: Carpmaels & Ransford LLP

(57) **Abstract**

A medical probe includes a proximal section, a transition region, and a distal section. The proximal section includes a braid that is braided with a cross-braiding configuration. In the transition region, which follows the proximal section, the braid transitions between the cross-braiding configuration and a helical coiling configuration. In the distal section, which follows the transition region, the braid is coiled with the helical coiling configuration.

## Description

### FIELD OF THE INVENTION

The present invention relates generally to medical probes, and particularly to catheter braiding configurations.

### BACKGROUND OF THE INVENTION

Medical probes, such as catheters, are used in a variety of therapeutic and diagnostic medical procedures. Various techniques for controlling the stiffness of a medical probe are known in the art. For example, U.S. Patent 6,152,912, whose disclosure is incorporated herein by reference, describes a catheter suitable for accessing a tissue target within the body, typically a target which is accessible through the vascular system. The catheter comprises a reinforcing member wound within the catheter body in such a way to create a catheter having a thin wall, kink-resistance, and controlled stiffness. As another example, U.S. Patent 6,258,080, whose disclosure is incorporated herein by reference, describes a surgical device such as a catheter. The catheter has stiffener ribbons, typically metallic, wound within the catheter body in such a way as to create a catheter having controllable stiffness.

U.S. Patent 5,037,404, whose disclosure is incorporated herein by reference, describes a flexible catheter comprising at least one resilient, flexible, tubular layer in telescoping relation with, and bonded to, a tubular wire sheath. A first catheter section includes the wire strands at a first angle to each other. A second catheter section includes the wire strands at a second angle to each other, with the second angle being different from the first angle so that the physical characteristics of the first and second catheter sections are different.

U.S. Patent 7,824,392, whose disclosure is incorporated herein by reference, describes a catheter braid formed from at least two continuous wires that are woven together. The catheter braid can include a proximal braid section and a distal braid section. Each of the continuous wires has a proximal diameter corresponding to the proximal braid section and a distal diameter corresponding to the distal braid section. The distal diameter of each wire can be less than the proximal diameter of that wire. A catheter braid so formed can be included in a catheter.

U.S. Patent 6,165,163, whose disclosure is incorporated herein by reference, describes a catheter assembly that may be used in accessing a tissue target within the body. of the catheter comprises a braided metallic reinforcing member, typically of a stainless steel or super-elastic alloy ribbon, situated within the catheter body in such a way to create a catheter section having a thin wall, controlled stiffness, and high resistance to kinking.

### SUMMARY OF THE INVENTION

An embodiment of the present invention provides a medical probe including a proximal section, a transition region and a distal section. The proximal section includes a braid that is braided with a cross-braiding configuration. In the transition region, which follows the proximal section, the braid transitions between the cross-braiding configuration and a helical coiling configuration. In the distal section, which follows the transition region, the braid is coiled with the helical coiling configuration.

In some embodiments, the braid in the proximal section includes multiple wires braided with the cross-braiding configuration, the braid in the distal section includes the multiple wires coiled with the helical coiling configuration, and in the transition region, a winding direction of a subset of the multiple wires is reversed so as to transition between the cross-braiding configuration and the helical coiling configuration.

In other embodiments, the medical probe includes an outer coating covering the braid. In yet other embodiments, the proximal section has a first flexibility in directions perpendicular to an axis of the probe, and the distal section has a second flexibility greater than the first flexibility.

In some embodiments, the probe includes a cardiac catheter. In other embodiments, the probe includes a cardiac sheath. In yet other embodiments, the probe includes a cardiac guide-wire.

In some embodiments, the braid in the proximal section, in the distal section and in the transition region is formed from a single unbroken set of wires. In other embodiments, the braid in the proximal section, in the distal section and in the transition region is formed from a single unbroken set of non-metallic filaments. In yet other embodiments, the braid in the proximal section, in the distal section and in the transition region is formed from a single unbroken set of non-metallic fibers.

There is also provided, in accordance with an embodiment of the present invention, a method including braiding a proximal section of a braid of a medical probe with a cross-braiding configuration. A transition region in the braid, following the proximal section, is produced by transitioning between the cross-braiding configuration and a helical coiling configuration. A distal section of the braid, following the transition region, is coiled using the helical coiling configuration.

The present invention will be more fully understood from the following detailed description of the embodiments thereof, taken together with the drawings in which:

### BRIEF DESCRIPTION OF THE DRAWINGS

Fig. 1 is a block diagram that schematically illustrates a catheter-based medical diagnostic system, in accordance with an embodiment of the present invention;
Fig. 2 is a diagram that illustrates a catheter with a cross-braided proximal section and a helically-coiled distal section, in accordance with an embodiment of the present invention; and
Fig. 3 is a flow chart that schematically illustrates a method for braiding a catheter, in accordance with an embodiment of the present invention.

### DETAILED DESCRIPTION OF EMBODIMENTS

### OVERVIEW

Medical probes such as catheters are used in a variety of diagnostic and therapeutic procedures, for example cardiac electrophysiological (EP) mapping and ablation. In such procedures, an operator of the procedure inserts the catheter percutaneously into the vascular system of a patient and navigates the catheter to a desired target region in the patient heart. The operator pushes and rotates the catheter in the vascular system of the patient until the distal end of the catheter reaches the target region. The maneuverability of the catheter in the cardio-vascular system is highly dependent on the flexibility of the catheter.

Embodiments of the present invention that are described herein provide medical probes, such as catheters, having improved maneuverability. In some embodiments, a catheter is formed using multiple braided wires that provide the desired mechanical stiffness. In a proximal section of the catheter, which typically extends through most of the catheter length, the wires are woven in a cross-braided configuration. In a distal section of the catheter, typically a short section next to the catheter tip, the wires are coiled in an unwoven, helical coiled (spiral) configuration. In a transition region between the proximal and distal sections, the wires transition between the cross-braided and helical-coiled configuration.

Both the cross-braided and helical-coiled configurations transmit torque around the catheter axis. Thus, the operator of the catheter is able to rotate the catheter about the catheter axis at its proximal end, and the rotation is transferred to the distal end. The helical coiling configuration, however, has greater flexibility in directions that are perpendicular to the catheter axis, relative to the cross-braiding section. Therefore, the distal section of the catheter is more flexible in these orthogonal directions than the proximal section, enhancing the overall maneuverability of the catheter.

Typically, the entire length of the catheter braid is formed from the same unbroken set of wires. In such embodiments, a first half of the wires from the proximal section continue to be wound in the same direction in the distal section. The second half of the wires change their winding direction to correspond to the winding direction of the first half, thus producing a dense helical coil. There is thus no change in the number of braid wires along the catheter.

### SYSTEM DESCRIPTION

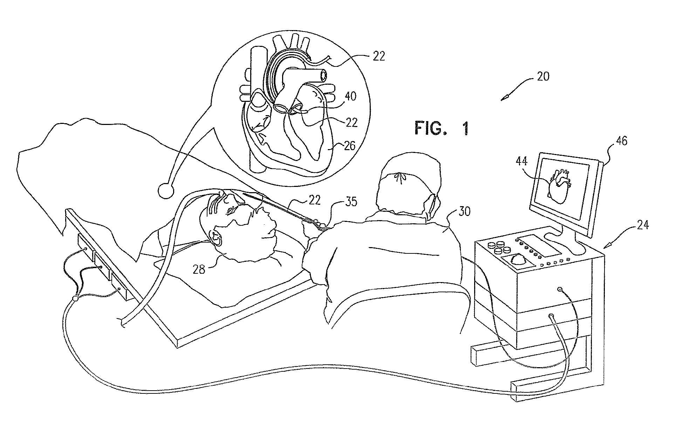

Fig. 1 is a block diagram that schematically illustrates a medical diagnostic system 20, in accordance with an embodiment of the present invention. System 20, in the present example a cardiac diagnostic system, comprises a catheter 22 and a control console 24. In the embodiment described herein, catheter 22 is used for cardiac diagnostic procedures such as measuring the electrophysiological (EP) signals at points on the surface of a cavity of a heart 26 of a patient 28 for the diagnosis of cardiac dysfunctions. Alternatively or additionally, catheter 22 may be used for other suitable therapeutic and/or diagnostic purposes, such as ablation of tissue in heart 26.

An operator 30, typically a physician, inserts catheter 22 into the vascular system of patient 28. A hand 35 of operator 30, holding the proximal end of catheter 22, navigates the catheter through the patient's vascular system until a distal end 40 of catheter 22 reaches the vicinity of the target tissue region in heart 26. The catheter contacts the tissue surface of the heart cavity to perform the cardiac diagnostic and/or therapeutic procedures.

Console 24 is configured to receive signals from electrodes disposed on catheter 22 and to control other components of system 20. System 20 may comprise a position tracking system to track the position of distal end 40 of catheter 22 in heart 26. Any suitable parameters may be measured and/or processed by system 20, and output on a display 46. For example, a mapping of an image 44 of heart 26 with local EP signals measured by electrodes on distal end 40 of catheter 22 contacting multiple points in the heart cavity may be output to display 46.

The embodiment shown in Fig. 1 is depicted merely for conceptual clarity, and not by way of limitation of the embodiments of the present invention. In alternative embodiments, any other suitable system configuration can be used. For example, the catheter may be manipulated in the vascular system of patient 28 by any suitable method, such as using machine-controlled navigation.

### IMPROVED MANUEVERABILITY USING DIFFERENT BRAIDING CONFIGURATIONS

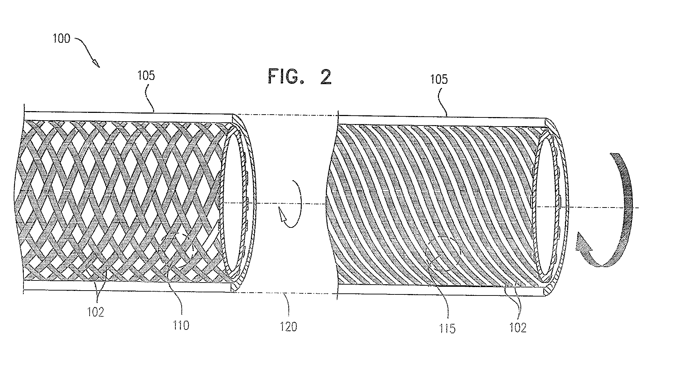

Fig. 2 is a diagram that illustrates a catheter 100, in accordance with an embodiment of the present invention. The proximal section of the catheter is shown on the left-hand-side of the figure, the distal section of the catheter is shown on the right-hand-side, and a transition region 120 is shown in the middle of the figure, connecting between the proximal and distal sections. The catheter, including the proximal section, transition region and distal section, is typically covered with a coating 105, e.g., a polyurethane or Silicone jacket.

The catheter typically comprises additional elements, such as signal wires traversing the internal lumen of the catheter, electrodes and/or sensors. These additional elements are omitted from the figure for the sake of clarity.

In the present example, the proximal section comprises a set of wires 102 that are woven in a cross-braiding configuration 110. In the distal section, the same set of wires 102 are coiled in a helical coiling (spiral) configuration 115. In transition region 120, the multiple wires change braiding configuration from cross-braiding configuration 110 to helical coiling configuration 115.

Typically, the distal section with helical coiling configuration 115 has greater flexibility than the proximal section with cross-braiding configuration 110, in directions that are perpendicular to the catheter axis.

Both sections transfer rotational torque about the catheter axis to the catheter distal tip, enabling operator 30 to rotate the catheter as desired. The cross-braiding configuration of the proximal section is able to transmit torque in both rotation directions (clockwise and counterclockwise). The helical coiling configuration of the distal section has a preferred rotation direction, which is shown using arrows in the figure.

Most of the catheter length comprises the proximal section with the stiffer cross-braiding configuration. In transition region 120, the multiple wires transition from cross-braided configuration 110 to the more flexible helical coiling configuration 115. Transition region 120 is typically close to distal end 40, but may generally be placed at any suitable position along the length of catheter 22, so as to optimize maneuverability in the patient's vascular system.

Transition region 120 in Fig. 2 is shown merely for visual clarity as a gap in the multiple wires woven between the proximal section to the distal section. The transition may be formed using any suitable transition between woven cross-braiding and unwoven helical coiling. For example, in some embodiments, transition region 102 comprises changing the winding direction of half of wires 102, and maintaining the winding direction of the other half. The number of wires 102 in this embodiment remains the same in transitioning from the proximal section with the cross-braiding configuration to the distal section with the helical spiral configuration.

In some embodiments, catheter 22 is formed using a conventional programmable controlled braider, such as a Steeger USA Braider Models No. K80/I6-IMC or HS80/I6-IMC, whose specifications are incorporated herein by reference. The braiding process typically begins by weaving the proximal section using cross-braided configuration 110. After a suitable length is braided, the weaving process is stopped and a coiler retrofit plate, such as Steeger Retrofit Kit Part No. STG04603.14, is manually attached to the braider. The braider is effectively converted into a coiler in which a horn gear turns sixteen bobbins on the retrofit plate in the same direction. The braiding process then continues to braid the distal section using helical coiling configuration 115.

In order to transition between braiding configurations, it is possible to retrofit the Steeger USA Braider with a retrofit plate that changes the direction of braiding elements (e.g., bobbins) to all rotate in the same direction. After installing the retrofit plate, the bobbins coil all of the multiple wires in the same direction to produce helical coiling configuration 115 of the distal section.

In other embodiments, the braider is programmed to engage the coiler retrofit plate, so as to automatically convert the braider into a coiler. The braiding configuration is changed from a cross-braiding to a helical coiling configuration to form transition region 120 without having to manually install the coiler retrofit plate on the braider.

Although the description above refers to a process that begins with cross-braiding and transitions to helical coiling, in alternative embodiments the process may be performed in the opposite direction, i.e., beginning with helical coiling and transitioning to cross-braiding.

Cross-braided configuration 110 shown in Fig. 2 comprises a diamond mesh formed by a first wire spiraling downward, which crosses over a second wire spiraling upward and then under a third wire spiraling upward, and so forth. This braid pattern is referred to herein as "one under one, over one".

The cross-braiding pattern shown in the embodiment of Fig. 2 is depicted purely for conceptual clarity and not by way of limitation of the embodiments of the present invention. Any other suitable cross-braided configuration can be used. For example, in alternative embodiments, a "one over two, under two" configuration may be used where a first wire spiraling downward crosses over a second and a third wire spiraling upward, and then under a third and a fourth wire spiraling upward, and so forth.

The braiding configurations described herein are not limited to constructing a catheter, but any suitable medical probe, such as a sheath or a guide-wire. Typically, the braid configurations shown in Fig. 2 used in the proximal section, distal section, and the transition region in the construction of the medical probe are formed from a single unbroken set of wires. Similarly, the medical probe can be constructed from braid configurations formed from a single unbroken set of non-metallic filaments, or non-metallic fibers.

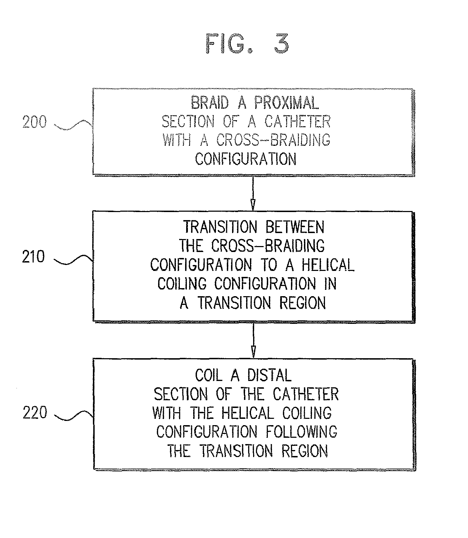

Fig. 3 is a flow chart that schematically illustrates a method for producing catheter 22, in accordance with an embodiment of the present invention. In a braiding step 200, the proximal section of catheter 22 is braided, or woven, with cross-braiding configuration 110. In a transitioning step 210, transition region 120 is produced, in which cross-braiding configuration 110 transitions into helical coiling configuration 115. In a coiling step 220, the distal section of catheter 22 is coiled with helical coiling configuration 115 following transition region 120 along the length of the catheter to the distal end.

It will thus be appreciated that the embodiments described above are cited by way of example, and that the present invention is not limited to what has been particularly shown and described hereinabove. Rather, the scope of the present invention includes both combinations and sub-combinations of the various features described hereinabove, as well as variations and modifications thereof which would occur to persons skilled in the art upon reading the foregoing description and which are not disclosed in the prior art. Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

## Claims

1. A medical probe, comprising:
a proximal section comprising a braid that is braided with a cross-braiding configuration;
a transition region, which follows the proximal section and in which the braid transitions between the cross-braiding configuration and a helical coiling configuration; and
a distal section, which follows the transition region and in which the braid is coiled with the helical coiling configuration.

2. The medical probe according to claim 1, wherein the braid in the proximal section comprises multiple wires braided with the cross-braiding configuration, wherein the braid in the distal section comprises the multiple wires coiled with the helical coiling configuration, and wherein, in the transition region, a winding direction of a subset of the multiple wires is reversed so as to transition between the cross-braiding configuration and the helical coiling configuration.

3. The medical probe according to claim 1, and comprising an outer coating covering the braid.

4. The medical probe according to claim 1, wherein the proximal section has a first flexibility in directions perpendicular to an axis of the probe, and wherein the distal section has a second flexibility greater than the first flexibility.

5. The medical probe according to claim 1, wherein the probe comprises a cardiac catheter, a cardiac sheath, or
a cardiac guide-wire.

6. The medical probe according to claim 1, wherein the braid in the proximal section, in the distal section and in the transition region is formed from a single unbroken set of wires, is formed from a single unbroken set of non-metallic filaments, or is formed from a single unbroken set of non-metallic fibers.

7. A method, comprising:
braiding a proximal section of a braid of a medical probe with a cross-braiding configuration;
producing a transition region in the braid, following the proximal section, by transitioning between the cross-braiding configuration and a helical coiling configuration; and
coiling a distal section of the braid, following the transition region, using the helical coiling configuration.

8. The method according to claim 7, wherein braiding the proximal section comprises braiding multiple wires with the cross-braiding configuration, wherein coiling the distal section comprises coiling the multiple wires with the helical coiling configuration, and wherein producing the transition region comprises reversing a winding direction of a subset of the multiple wires so as to transition between the cross-braiding configuration and the helical coiling configuration.

9. The method according to claim 7, and comprising applying an outer coating to cover the braid.

10. The method according to claim 7, wherein the proximal section has a first flexibility in directions perpendicular to an axis of the probe, and wherein the distal section has a second flexibility greater than the first flexibility.

11. The method according to claim 7, wherein the probe comprises a cardiac catheter, a cardiac sheath, or a cardiac guide-wire.

12. The method according to claim 7, wherein braiding the proximal section, coiling the distal section, and producing the transition region comprise forming the braid in the proximal section, in the distal section and in the transition region from a single unbroken set of wires, from a single unbroken set of non-metallic filaments, or from a single unbroken set of non-metallic fibers.
